(19) Europäisches Patentamt / European Patent Office / Office européen des brevets

(11) **EP 3 235 533 A1**

(12) **EUROPEAN PATENT APPLICATION**

(43) Date of publication:
25.10.2017 Bulletin 2017/43

(51) Int Cl.:
*A61M 15/00* *(2006.01)*

(21) Application number: 17171252.4

(22) Date of filing: 06.10.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 07.10.2010 TR 201008226
04.10.2011 TR 201109804

(62) Document number(s) of the earlier application(s) in
accordance with Art. 76 EPC:
**11799517.5 / 2 624 898**

(71) Applicant: Bilgic, Mahmut
34220 Esenler-Istanbul (TR)

(72) Inventor: Bilgic, Mahmut
34220 Esenler-Istanbul (TR)

Remarks:
This application was filed on 16-05-2017 as a
divisional application to the application mentioned
under INID code 62.

(54) **SINGLE DOSE DRY POWDER INHALATION DEVICE**

(57) The present invention relates to an inhaler which is suitable for administration of drugs in dry powder form used in the prophylaxis and the treatment of respiratory tract diseases from capsules and enables inhalation of the medicament easily even at low flow rates in order to ensure an effective inhalation.

## Figure 9a

EP 3 235 533 A1

## Description

The present invention relates to an inhaler suitable for administration of drugs in dry powder form used in the prophylaxis and the treatment of respiratory tract diseases by the inhalation route.

The inhalers in which the dry powder medicament is carried in blister packs or capsules can be multiple-dose or single-dose inhalers. The term "multiple-dose inhaler" refers to inhalers comprising more than one medicament dose. The term "single-dose inhaler", on the other hand, refers to inhalers comprising a single medicament dose.

The inhalers enabling the inhalation of dry powder medicament from capsules are commonly used. In general, one capsule is placed in the capsule chamber of the device before each inhalation in these devices; the capsule in the capsule chamber of the device is pierced, cut or opened by similar methods when the device is actuated, and the dry powder medicament contained in the capsule becomes ready for inhalation.

Since the active agents used in inhalation treatment induce rather strong effects, the amount of the active agent in one dose of dry powder medicament is quite low. Thus, absorption of a sufficient amount of the active agent in the patient's lung is essential in order to realize an effective inhalation. Absorption of less than the required amount of the active agent in the patient's lung remains insufficient for an effective treatment.

The inhaler marketed under the trade mark Spiriva® Handihaler® by Boehringer and Pfizer is one of the inhalers available on the market. Although this inhaler is a commonly used inhalation device on the market, there is still need for devices having different mechanisms and specifications so as to realize an effective inhalation.

In order to deliver sufficient dry powder medicament to the lungs, it is essential to ensure a minimum pressure decrease of 4 kPa in the inhaler during inhalation. Thus, analyses about inhalers are carried out on this basis. However, ensuring this pressure decrease in the inhaler during the inhalation depends on resistance of the inhaler and flow rate applied by the patient. In the cases that device resistance is low, the flow rate required to be applied by the patient must be high in order to ensure a minimum pressure decrease of 4 kPa in the inhaler during inhalation. This inverse proportion between the resistance of the inhaler and the flow rate is shown in the equation below:

$$\sqrt{\Delta P} = Rd.Q$$

In the equation, "$\Delta P$" represents the pressure decrease required to be ensured in the inhaler for an effective inhalation and its minimum value is 4kPa. "Rd" and "Q" in the equation represent the resistance of the inhaler and the flow rate that the patient has to apply during the inhalation respectively. The fact that the flow rate that the patient has to apply is high requires the patient to spend more effort during the inhalation to ensure the minimum pressure decrease of 4 kPa in the inhaler. Since the maximum flow rate that each patient can apply varies, not all patients can achieve to use these inhalers having low resistance and the medicament cannot be delivered to the patients lungs in an effective amount for the treatment.

The inventor has surprisingly found that the ratio of the diameter of needles piercing the capsule to the diameter of the holes through which the needles pass and enter the capsule chamber in order to pierce the capsule affects the resistance of the inhaler to a substantial extent in an inhaler comprising capsule.

From a general perspective, the present invention relates to an inhaler which comprises capsule and enables effective inhalation of dry powder medicament even at low flow rates.

In detail, the inhaler of the present invention which comprises capsule and used so as to deliver dry powder medicament comprises a mouthpiece cover hiding the mouthpiece; a bottom casing where the device mechanism is situated; a movable mouthpiece communicating with the bottom casing; a middle casing situated between the bottom casing and the mouthpiece; a capsule chamber where the capsule is placed characterized in that the ratio of the diameter of needles piercing the capsule that comprises medicament in dry powder form before inhalation to the diameter of the holes through which the needles pass and enter the capsule chamber in order to pierce the capsule is in the range of 0.1 to 1.0, for instance in the range of 0.1, 0.2, 0.3, 0.4, 0.5 to 0.51, 0.6, 0.7, 0.8, 0.9, 1.0.

In another aspect, an inhaler of the present invention mentioned above characterized in that the ratio of the diameter of needles piercing the capsule that comprises medicament in dry powder form before inhalation to the diameter of the holes through which the needles pass and enter the capsule chamber in order to pierce the capsule is preferably in the range of 0.25 to 1.0, for instance in the range of 0.25, 0.30, 0.35, 0.4, 0.45, 0.5 to 0.51, 0.55, 0.6, 0.65, 0.70, 0.75, 0.80, 0.85, 0.90, 0.95, 1.00; more preferably in the range of 0.3 to 1.0.

The flow rate required to ensure the pressure decrease of 4 kPa in the inhaler for an effective inhalation must be applied by the patient. In the inhaler of the present invention, it has been found that the distance between the needles piercing the capsule and the holes through which the needles pass and enter the capsule chamber in order to pierce the capsule affects the resistance of the inhaler and therefore the flow rate required to ensure the minimum pressure decrease of 4 kPa to a substantial extent. Some of the air flow entering the device upon inhalation of the patient enters the capsule chamber through the gap between the needles and the needle holes. An increase or decrease in the distance between the needles and the needle holes, in other terms, an increase or decrease in the ratio of the diameter of the needles to the diameter of the needle holes affects the cross-sectional area through which the air flow entering the capsule chamber through the gap between the needles and the needle holes passes; therefore the rate of said air flow to a substantial extent. A decrease in the cross-sectional area through which said air flow passes results in an increase in air flow rate; an increase in the cross-sectional area through which said air flow passes results in a decrease in air flow rate. Increase of air flow rate results in increase of friction caused during air's pass between the needles and the needle holes and therefore increase in resistance of the inhaler. Increase of resistance of the inhaler induces a decrease in flow rate which is required to be applied by the patient in order to attain to the minimum pressure decrease of 4 kPa in the inhaler. In the case that the ratio of diameter of needles piercing the capsule containing dry powder medicament before inhalation to diameter of needle holes through which the needles pass and enter the capsule chamber in order to pierce the capsule is in the range of 0.1 to 1.0 in the inhaler designed by the inventor, the flow rate that must be applied in order to ensure minimum pressure decrease of 4 kPa can easily be applied by almost all patients at different ages and strength.

Furthermore, the ratio between diameter of the needles and diameter of the needle holes also affects the turbulence caused in the capsule chamber during inhalation. Thus, in the case that the ratio of diameter of the needles to diameter of needle holes is in the range of 0.1 to 1.0, the turbulence created in the capsule chamber during inhalation provides to disaggregate agglomerates of the dry powder medicament formed in the capsule, and prevent adhesion of dry powder particles to inner surface of the capsule chamber due to electrostatic forces. In addition to these, the turbulence created in the capsule chamber during inhalation enables the medicament in dry powder form to be inhaled with appropriate particle size distribution.

The term "resistance of the inhaler" refers to resistance of the inhaler against air flow.

The term "flow rate" refers to volume of air flow that the patient must ensure per minute during the inhalation of the medicament in dry powder form in the inhaler.

In another aspect, the present invention provides an inhaler which enables to deliver a sufficient amount of the dry powder medicament used in the treatment of respiratory diseases to the patient's lungs.

In the inhaler of the present invention, diameter values of the needle holes through which the needles pass and enter the capsule chamber in order to pierce the capsule can be identical or different though diameter values of said needle holes are preferably identical.

In the inhaler of the present invention, diameters of the needles can be identical or different though diameter values of said needles are preferably identical.

In the inhaler of the present invention, number of needle holes through which the needles pass and enter the capsule chamber in order to pierce the capsule can be at least two though it is preferably two. Said needle holes of the inhaler of the present invention are hollow-cylindrical such that the needles can pass through them.

In the inhaler of the present invention, the needles piercing the capsule are cylindrical and the points of the needles which enable to pierce the capsule are sharp in any shape. The capsule comprising medicament in dry powder form can easily be pierced with the effect of strong pressure that the needles apply thanks to their sharp points.

In the inhaler of the present invention, the needle holes through which the needles pass and enter the capsule chamber in order to pierce the capsule can be situated diagonally, symmetrically, asymmetrically, side by side and/or vis-à-vis on a horizontal or vertical line, at equal and/or different intervals.

These needles ensuring the capsule to be pierced during inhalation can be made of any suitable metal and/or nonmetal material though they are preferably made of stainless steel. In addition, these needles can be coated with any suitable coating material in order to give a smooth and bright look though they are preferably coated with chrome, nickel, brass and/or a combination thereof.

In another aspect, a sufficient amount of active agent has to be absorbed in the lungs for an effective inhalation to be realized. Delivery and absorption of a sufficient amount of active agent in the lungs of the patient is possible when the medicament in dry powder form that is inhaled has a uniform and homogeneous particle size distribution. However, in the dry powder medicament in the capsule, agglomerates are formed due to factors such as moisture rate of the capsule, electrostatic forces, energy exchange between dry powder particles, etc. These agglomerations negatively affect the particle size distribution of the medicament in dry powder form in the capsule. Inhalation of a dry powder medicament which does not have a uniform or homogenous particle size distribution results in failure to deliver a sufficient amount of active agent to the lungs and its absorption there.

The particle size of the active agent which can be absorbed in the lungs is in the range of 1 $\mu$m to 6 $\mu$m. Other particles having larger particle size in the dry powder medicament play a significant role in entraining the active agent from the inhaler to the patient's lungs. Coarse particles may get caught in oral cavity, throat and bronchial tube before reaching the patient's lungs during inhalation. Since the agglomerates formed in the dry powder medicament in the capsule may be in large sizes and comprise some active agent, they conduce to failure in delivery of a sufficient amount of active agent in the lungs and its absorption there. To this respect, these agglomerates formed in the medicament in dry powder form have to be disaggregated in the inhaler before delivered to the patient.

The inventor has surprisingly found that turbulence is created in the air flow exiting the capsule chamber which entrains dry powder medicament and the formulation is inhaled with a uniform and homogenous particle size distribution and therefore a sufficient amount of active agent is delivered to the lungs due to this turbulence in the case that there are at least two holes at the outlet of the capsule chamber of the inhaler comprising capsule having identical or different diameters. In another aspect, the inventor has designed an inhaler which enables delivery of medicament in dry powder form with uniform and homogenous particle size distribution to the patient in order to solve the problems above.

In detail, the inhaler of the present invention which comprises capsule containing dry powder medicament comprises a mouthpiece cover hiding the mouthpiece; a bottom casing where the device mechanism is situated; a movable mouthpiece communicating with the bottom casing; a middle casing situated between the bottom casing and the mouthpiece; a capsule chamber where the capsule is placed characterized in that there are at least two holes having identical or different diameters of at least 1 $\mu$m situated at the outlet of the capsule chamber, in the area where the capsule chamber is at the center, through which some part of the air flow entering said inhaler passes during inhalation and provides to disaggregate the agglomerates formed in the medicament in dry powder form in the capsule at the outlet of the capsule chamber before the medicament is delivered to the patient.

These holes at the outlet of the capsule chamber of the inhaler of the present invention ensure to create turbulence in the air flow which exits the capsule entraining medicament in dry powder form; therefore to disaggregate the agglomerates formed in the medicament in dry powder form before delivery to the patient. The air flow passing through these holes at the outlet of the capsule chamber, in the area where the capsule chamber is in the center, affects the flow direction of the particles of the dry powder medicament; therefore it creates turbulence in the air flow entraining dry powder medicament at the outlet of the capsule chamber. The turbulence created in the air flow entraining dry powder medicament conduces to pressure increase at the outlet of the capsule chamber, in the area where the capsule chamber is in the center. So, the pressure here gets higher compared with the pressure at the outlet of the mouthpiece and this pressure difference eases delivery of the air flow entraining medicament in dry powder form since the medicament in dry powder form moves from high pressure to low pressure in general. In addition, due to the turbulence created in the air flow entraining dry powder medicament, agglomerates in the medicament in dry powder form are disaggregated and the dry powder medicament entrained by the air flow is delivered to the patient with a uniform and homogenous particle size distribution.

In the studies conducted in scope of the present invention, it has been observed that an effective turbulence is created in the air flow entraining the dry powder medicament from capsule to the patient in the case that the diameter of each one of at least two holes situated at the outlet of the capsule chamber, in the area where the capsule chamber is in the center, is in the range of 1 $\mu$m to 1000 $\mu$m, preferably in the range of 1 $\mu$m to 750 $\mu$m; for instance in the range of 1, 2, 3, 5, 6, 7, 8, 9, 10, 12, 14, 16, 18, 20 $\mu$m to 550, 600, 650, 700, 750 $\mu$m; more preferably in the range of 1 $\mu$m to 500 $\mu$m, for instance in the range of 1, 2, 3, 4, 5, 6, 7, 8, 9, 10, 12, 14, 16, 18, 20, 25, 30, 40, 50 $\mu$m to 300, 350, 400, 450, 500 $\mu$m.

The fact that the holes at the outlet of the capsule chamber of the inhaler of the present invention, in the area where the capsule chamber is in the center, create turbulence in the air flow entraining dry powder medicament from the capsule depends on the flow rate applied by the patient and the cross-sectional area of the holes through which the air flow entraining medicament particle passes. This correlation is explained with Reynolds number formula shown in the formula below:

$$N_{Re} = \frac{d v \rho}{\mu}$$

In order for the holes at the outlet of the capsule chamber of the inhaler of the present invention, in the area where the capsule chamber is in the center, to create turbulence in the air flow entraining dry powder medicament particles from the capsule, the dimensionless Reynolds number ($N_{Re}$) calculated with the formula above must be greater than 4000 for the air flow passing through each hole. According to this, the turbulence in the air flow passing through each hole depends on Reynolds number represented with "$N_{Re}$" and therefore on parameters such as diameter represented with "d" through which the air flow passes; flow rate represented with "v"; air density represented with "p" and air viscosity represented with "$\mu$". As it is seen in the formula, when it is taken into consideration that air density and viscosity are stable, it is seen that Reynolds number, therefore flow rate of turbulence change (v) depends on the cross-sectional area through which the air flow passes therefore the diameter (d), and there is an inverse proportion between these two parameters. In the studies conducted in scope of the present invention, the smaller the diameter of any holes at the outlet of the capsule chamber in the area where the capsule chamber is in the center, the faster the air flow rate passing through the hole; thus the more the turbulence is. It was also seen that turbulence completely disappears when the diameter value of any hole at the outlet of the capsule chamber, in the area where the capsule chamber is in the center drops down below a certain value. This situation is explained this way: the smaller the diameter of the hole gets, the faster the flow rate is, and turbulence is caused as Reynolds number exceeds the value of 4000. However, when the diameter value of the hole drops down below a certain level, the flow rate provided does not suffice to put the Reynolds number over the value of 4000 and laminar (not turbulent) air flow is created. Similarly, turbulence disappears also in the cases that the diameter of the hole goes up above a certain value. To this respect, in order to ensure creation of turbulence in the inhaler, it is required that the holes situated at the outlet of the capsule chamber, in the area where the capsule chamber is in the center, must have a certain diameter value. In the inhaler designed by the inventor, a sufficient amount of active agent can be delivered to the patient's lungs and an effective inhalation is realized when the diameter of the holes situated at the outlet of the capsule chamber, in the area where the capsule chamber is in the center, is in the range of 1 $\mu$m to 1000 $\mu$m.

The term "Reynolds number, $N_{Re}$" refers to dimensionless number that quantifies the relative importance of inertial forces ($dv\rho$) to viscous forces ($\mu$) for certain flow conditions during inhalation.

The term "laminar flow" refers to flow occurring when the air flow entraining the dry powder medicament in the inhaler flows in parallel layers, with no disruption between the layers.

The term "flow rate" refers to the volume of air flow that the patient should provide per minute during inhalation of the dry powder medicament in the inhaler.

The term "effective inhalation" refers to an inhalation in which a sufficient amount of active agent required for an effective treatment is delivered to the patient's lungs.

After the capsule in the capsule chamber of the inhaler of the present invention is pierced, while some of the external air entering the device upon the inhalation of the patient in order to inhale the dry powder medicament in the capsule enters into the capsule chamber, entrains the medicament in dry powder form from the pierced capsule and then from the capsule chamber towards the mouthpiece, the rest of the air passes through the holes at the outlet of the capsule chamber, in the area where the capsule chamber is in the center, and create turbulence in the air flow entraining dry powder medicament at the outlet of the capsule chamber. In the case that the diameters of these holes at the outlet of the capsule chamber, in the area where the capsule chamber is in the center, are different from each other, the flow rates of the air passing though each hole are also different. In the case that the diameters of these holes at the outlet of the capsule chamber, in the area where the capsule chamber is in the center, are identical, the flow rates of the air passing though each hole are also the same. The diameters of the holes at the outlet of the capsule chamber, in the area where the capsule chamber is in the center, can be different though they are preferably the same in order to ensure an effective turbulence.

The term "an effective turbulence" refers to turbulence which ensures to disaggregate all agglomerates formed in the medicament in dry powder form entrained by the air flow entering the capsule chamber of the inhaler upon inhalation of the patient.

The holes which are situated at the outlet of the capsule chamber, in the area where the capsule chamber is in the center, and preferably have identical diameters prevent accumulation of medicament particles at the outlet of the capsule chamber and thus at the inlet of the air intake duct due to electrostatic forces in addition to providing to disaggregate the agglomerates in the medicament in dry powder form entrained by the air flow entering the capsule chamber. After the air flow entraining the medicament in dry powder form exits the capsule chamber, it passes through the air intake duct and the mouthpiece of the inhaler and reaches the patient.

Number of the holes at the outlet of the capsule chamber, in the area where the capsule chamber is in the center, is at least two though it is preferably three. The number of said holes can change depending on the diameter of the holes.

The holes at the outlet of the capsule chamber, in the area where the capsule chamber is in the center, can be situated at any suitable position though they are preferably situated such that the angle between said holes is equal.

The inhaler of the present invention comprises preferably a pierceable capsule which comprises medicament in dry powder form.

The inhaler of the present invention is composed of various components in order to ensure inhalation of the medicament in dry powder form contained in the capsule.

According to this, basic components of the inhaler are the mouthpiece cover, bottom casing, capsule chamber, mouthpiece and the middle chasing. The mouthpiece cover covers the mouthpiece and provides to keep it clean. The mouthpiece which is exposed when the mouthpiece cover is opened is joined with the middle casing and they synchronize. In the bottom casing, the capsule containing the medicament in dry powder form and the mechanism of the device are situated. On the sides of the middle casing, there are holes used as pin holders. The pins placed in these pin holders enables the mouthpiece cover, mouthpiece, middle casing with the mouthpiece, capsule chamber and the bottom casing to easily move around the pin they are attached to.

Mechanism of the device is situated in the bottom casing of the inhaler. The mechanism is composed of components which contribute to the inhaler of the present invention to work properly and the medicament in dry powder form contained in the capsule to be prepared for inhalation. The piercing needles which pierce the capsule; the needle carrier; the springs which enable the needle carrier to move towards and away from the capsule chamber and to be positioned correctly according to the needles and to remain fixed at this position are among components constituting the mechanism of the device.

There are holes on the capsule chamber in which the capsule containing dry powder medicament is placed in order to enable the needles to enter and exit the compartment where the capsule is placed. The needles pass through this hole and pierce the capsule. The connecting rod in the capsule chamber is also inserted into the spring and the hole in the center of the needle carrier between the capsule chamber and the needle carrier and provides said spring to be positioned on it. According to this, said spring is situated between the capsule chamber and the needle carrier. The other spring in the mechanism of the device is on the other side of the needle carrier, between the needle carrier and the inner surface of the bottom casing. In other words, the needle carrier is situated between the two springs.

At the end of the air intake duct in the capsule chamber side, there placed a sieve and a sieve holder above this sieve providing to keep it fixed. The sieve holder provides to keep the sieve embedded with the air intake duct. The air intake duct inserted into the mouthpiece is also fixed into this sieve. Therefore, the air exiting the capsule chamber passes through the sieve, the sieve holder and the air intake duct before being delivered to the patient as the air intake duct is on the same axis with the sieve and the sieve holder. Furthermore, there are the holes mentioned above at the outlet of the capsule chamber in order to create turbulence in the air flow entraining dry powder medicament during the inhalation. In addition to these, the capsule chamber cover covering the capsule chamber provides to protect the capsule chamber and the mechanism of the device from external factors.

Prior to inhalation, the mouthpiece which synchronizes preferably with the middle casing has to be rotated outwards around the pin it is joined with in order to place the capsule containing the medicament in dry powder form in the capsule chamber. This movement of the mouthpiece is maintained until the capsule chamber gets to an available position for placing a capsule. For the capsule to be pierced after being placed in the capsule chamber, the mouthpiece has to be moved towards the capsule chamber. During the move of the mouthpiece towards the capsule chamber, while the pawls at the ends of the cogs reaching down under the mouthpiece are advancing on the rails in both sides of the needle carrier, the needle carrier compresses the spring and the piercing needles are moved towards the capsule. Furthermore, when each of the pawls at the ends of the cogs reaching down under the mouthpiece gets close to the corner of the rail it advances on, the spring approaches to maximum compression as the needle carrier is moved towards the capsule chamber and the needles pierce the capsule passing through the holes on the capsule chamber. While this movement of the mouthpiece is continuing, the pawls' passing the corner causes the spring to loosen and therefore the needle carrier to move away from the capsule chamber. When the mouthpiece is completely closed, the needle carrier is in the accurate and exact position between the two springs; and the needles are out of the capsule. In this position of the mouthpiece, medicament in dry powder form contained in the pierced capsule is ready for inhalation.

The air flow entering the device upon inhalation of the patient goes through the holes of the capsule in the capsule chamber and entrains the dry powder medicament in the capsule out. Subsequently, the air flow entraining the dry powder medicament ensures the delivery of the medicament in dry powder form to the patient passing through the sieve, sieve holder and the air intake duct.

The mouthpiece is moved outwards after the dry powder medicament in the pierced capsule is inhaled by the patient and removed from the capsule chamber. In the case that the mouthpiece moves with the middle casing, the capsule chamber is exposed when the mouthpiece is moved outwards around the pin it is attached to. Thus, the discharged capsule is ejected from the capsule chamber after the inhalation and the capsule chamber is cleaned properly if required. The mouthpiece and the mouthpiece cover are kept close until the next inhalation.

The inhalation device of the present invention comprising capsule can be made of the same or different materials. According to this, each component of the inhaler can be made of a suitable material though it is preferably selected from a group comprising styreneacrylonitrile, polyoxymethylene, acrylic polymethyl metacrylate, cellulose acetate, polyetheretherketone, polyvinyl chloride, polyethylene, polypropylene, acrylonitrile butadiene styrene, silicon, polycarbonate, polyamide, polystyrene, polyurethane or fluoropolymer types. Furthermore, each component of the device can be in any suitable color.

The capsule used in the inhaler comprising a capsule pertaining to the present invention can be made of any suitable substance though it is preferably made of a material selected from a group comprising gelatin, chitosan, starch and/or starch derivatives, cellulose and/or cellulose derivatives or synthetic polymers. In addition, the capsule of the present invention is composed of intertwining top and bottom compartments. The top and the bottom compartments of said capsule can be made of identical or different materials.

According to this, in the case that the capsule used in the present invention is made of cellulose or its derivatives, the capsule material can be selected from, but not limited to, a group comprising hydroxypropyl cellulose, hydroxypropylmethyl cellulose, methyl cellulose, hydroxymethyl cellulose, hydroxyethyl cellulose.

In the case that the capsule used in the present invention is synthetic polymer, the capsule material can be selected from, but not limited to, a group comprising polyethylene, polyetheleneteraphtalate, polycarbonate or polypropylene.

In the case that the capsule material used in the present invention is gelatine, additional agents such as polyethylene glycol, sorbitol, glycerol, propylene glycol, polyethylene oxide - polypropylene oxide block copolymers and/or other polyalcohols and polyethers at different molecular weights can be added into it.

The volume of the capsule containing the dry powder medicament in the inhaler of the present invention is in the range of 0.10 ml to 0.70 ml, preferably in the range of 0.10 ml to 0.52 ml and it is filled up to 0.01-30 %, preferably 0.01-25 % of said volume.

Moreover, the capsule pack containing the dry powder medicament and used in the inhaler of the present invention can be in any suitable shape and color on condition that it holds the specifications mentioned above.

The drawings given in the appendix in order to exemplify the present invention are explained with the reference numbers below. The drawings aim to exemplify the invention, yet the invention cannot be limited to these drawings and descriptions.

Figure 1 is a front view of the inhaler of the present invention.

Figure 2 is an exploded view of the inhaler of the present invention.

Figure 3 is a view of the communication of the main components of the device of the present invention.

Figures 4a and 4b are views of the capsule chamber with the needle carrier of the inhaler of the present invention.

Figure 5 is a view of route that the mouthpiece of the inhaler of the present invention follows during its move through the capsule chamber.

Figure 6 is a view of the capsule chamber with the mouthpiece when the mouthpiece is entirely covered.

Figure 7 is a view of the capsule chamber with the mouthpiece and the middle casing when the mouthpiece is entirely covered.

Figure 8 is a view of the positions of the needles and the needle holes of the inhaler of the present invention to each other.

Figures 8a-8b are bottom and side views of the mouthpiece of the inhaler of the inhaler respectively.

Figures 9a-9b are plan and bottom views of the capsule chamber of the inhaler respectively.

Figure 10 is a vertical section view of the inhaler of the present invention.

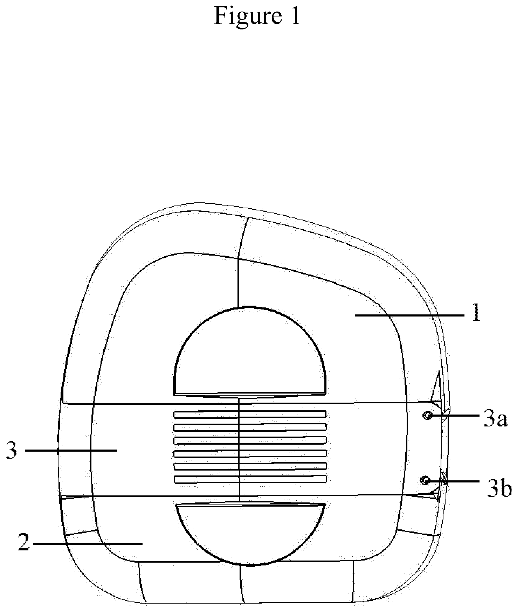

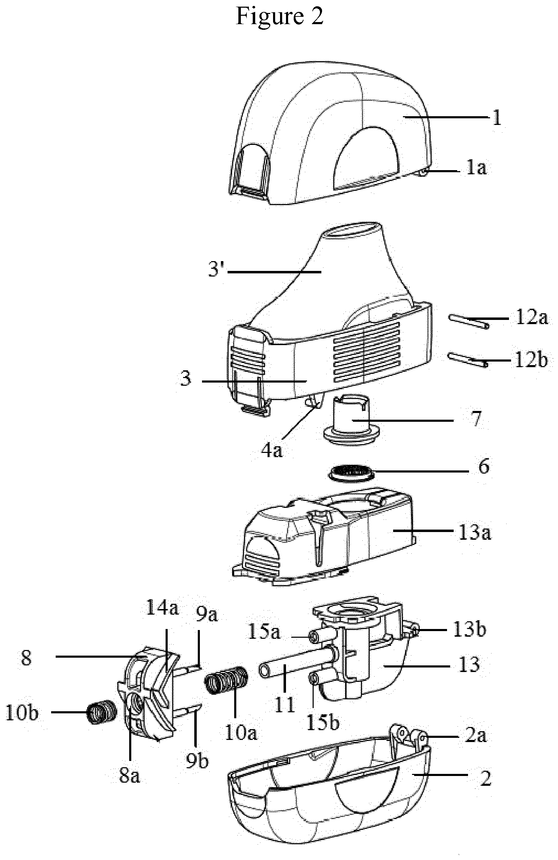

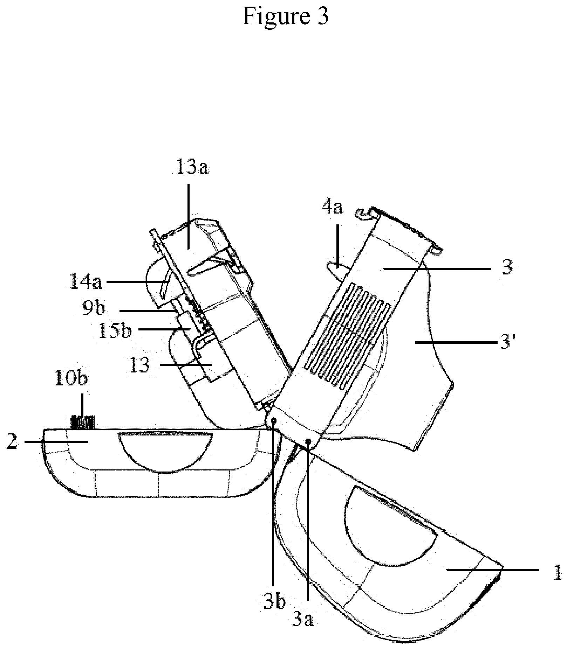

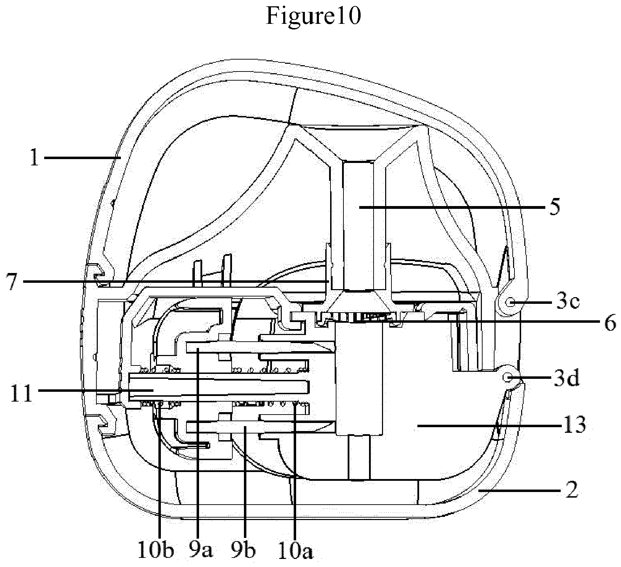

The preferred embodiment of the inhaler of the present invention is as in Figure 1 and Figure 2 in the appendix though the components of the inhaler of the present invention are illustrated in figures 3 to 10.

The inhaler of the present invention illustrated in Figure 1 is in pre-use position. In pre-use position, only the mouthpiece cover (1) hiding the mouthpiece; the bottom casing (2) where the mechanism of the device is situated; and the middle casing (3) are visible. The mouthpiece cover (1) covers the movable mouthpiece (3') component which is connected with the bottom casing (2) of the inhaler in pre-use position. Therefore, the mouthpiece cover (1) enables to keep the mouthpiece (3') clean in pre-use position and carry it everywhere comfortably.

Each component of the inhaler and their communication with each other can be clearly seen in the exploded view of the inhaler of the present invention in Figure 2. The mechanism of the inhaler of the present invention and the capsule chamber (13) are situated in the bottom casing (2) of the device. The mechanism of the inhaler is composed of constituents which contribute to ensure proper operation of the inhaler of the present invention and prepare the medicament in dry powder form contained in the capsule of the inhaler for inhalation. The piercing needles (9a, 9b) that pierce the capsule, the needle carrier (8), the springs (10a, 10b) which enable the needle carrier to easily move towards and away from the capsule chamber and keep the needle carrier at the accurate position according to the positions of the needles are among the constituents composing the mechanism of the inhaler. According to figure 2, the mouthpiece (3') and the middle casing (3) are joined and they synchronize. The capsule chamber cover (13a) which covers the capsule chamber (13) ensures protection of the capsule chamber (13) and the mechanism of the inhaler from external factors. There is a sieve (6) on the outlet of the capsule chamber (13) and a sieve holder (7) which keeps this sieve fixed.

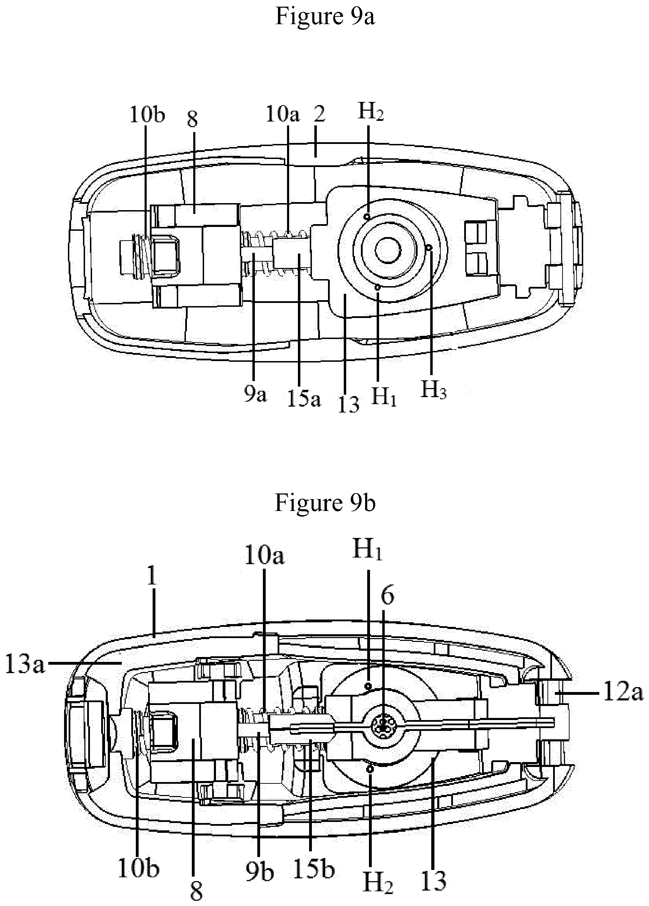

In figure 3, the mouthpiece cover (1), the bottom casing (2), the middle casing (3) and the mouthpiece (3') which are the main components of the inhaler and the positions of each around the pins (12a, 12b) they are attached to are illustrated. According to figure 3 and 9b, the upper pin (12a) and the lower pin (12b) are kept in a precise position by the pin holders (3a, 3b, 3c, 3d) on the middle casing (3). The upper pin (12a) is hold by the pin holders (3a, 3c) on the upper part of the middle casing; the lower pin (12b) is hold by the pin holders (3b, 3d) on the lower part of the middle casing. The mouthpiece cover (1) of the inhaler of the present invention is joined with the upper pin (12a); the bottom casing (2), the middle casing (3), the mouthpiece (3') and the capsule chamber (13) are joined with the lower pin (12b), and these components can easily be rotated around the pins they are joined with. The mouthpiece cover (1) is joined with the upper pin (12a) via the mouthpiece pin hole (1a); the bottom casing (2) and the capsule chamber (13) are joined with the lower pin (12b) via the bottom casing pin hole (2a) and the capsule chamber pin hole (13b) respectively.

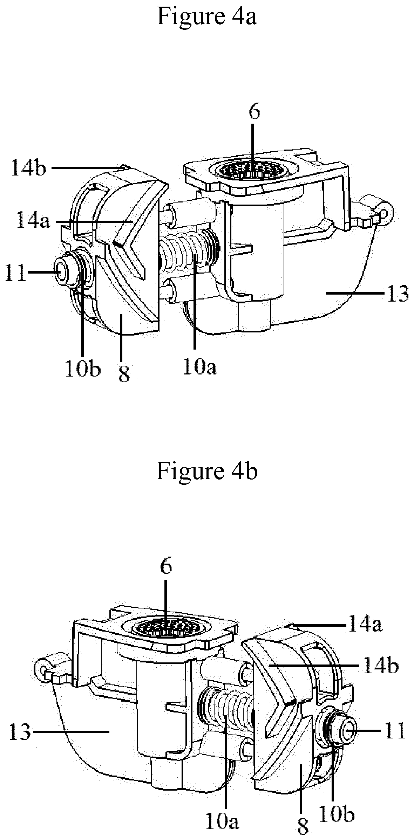

In figures 4a-4b, the needle carrier (8) is illustrated with the capsule chamber (13). There are holes (15a, 15b) on the capsule chamber (13) where the capsule comprising the medicament in dry powder form is placed in order to enable the needles (9a, 9b) to go in and out of the compartment where the capsule is. The needles (9a, 9b) held by the needle carrier (8) go through these holes (15a, 15b) on the capsule chamber (13) and pierce the capsule. The connection rod (11) in the capsule chamber (13) goes into the hole (8a) in the center of the needle carrier (8) with the spring (10a) between the capsule chamber (13) and the needle carrier (8). The other spring (10b) in the mechanism of the inhaler is on the other side of the needle carrier (8), between the needle carrier (8) and the inner surface of the bottom casing (2). In other words, the needle carrier (8) is situated between the two springs (10a, 10b). This spring (10b) on the other side of the needle carrier (8) enables accurate and exact positioning of the needle carrier (8) when the mouthpiece (3') is completely closed.

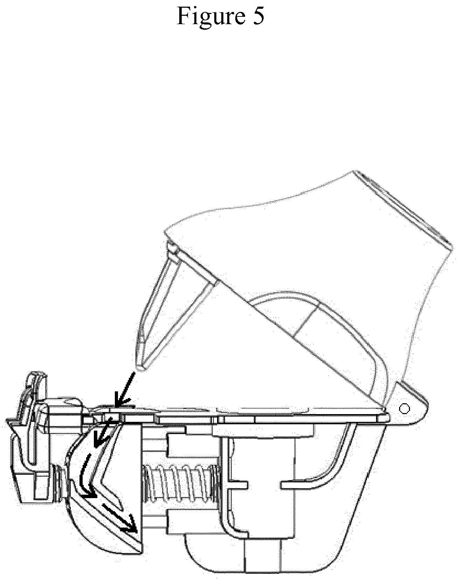

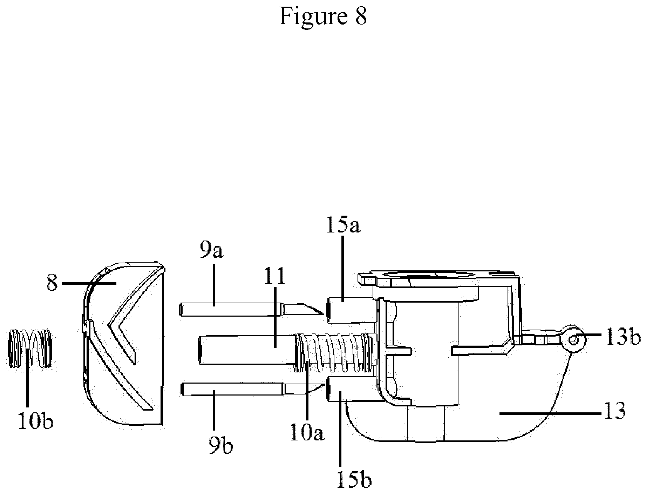

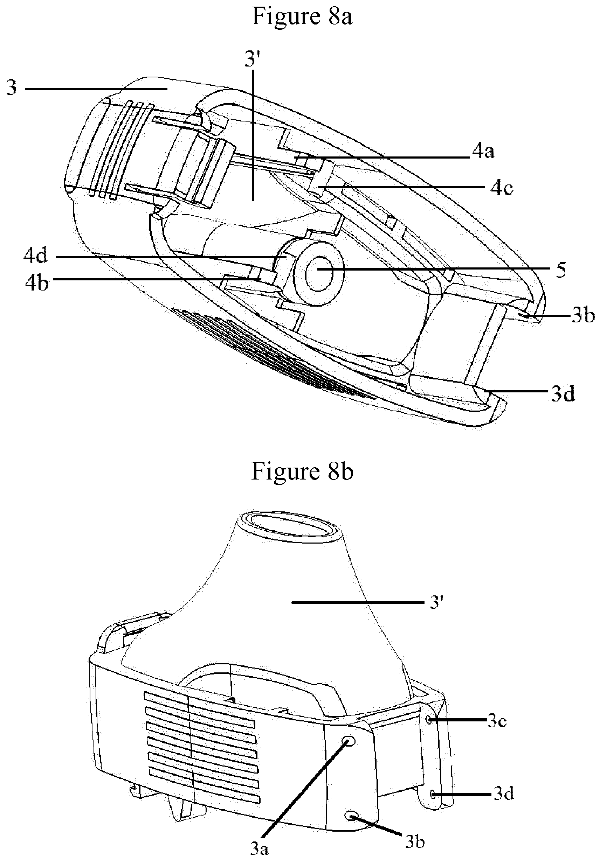

Movement of the mouthpiece (3') towards the capsule chamber (13) after the capsule is placed in the capsule chamber (13) is illustrated in figures 5, 6 and 7. The mouthpiece (3') is advanced on the rails (14a, 14b) on both sides of the needle carrier (8) thanks to the pawls (4c, 4d) at the ends of the cogs (4a, 4b) reaching down under the mouthpiece (3'). In more detail, while one of the pawls (4c) at the ends of the cogs reaching down under the mouthpiece (3') is advancing on the path (14a) in its side, the pawl (4d) at the end of the other cog reaching down under the mouthpiece (3') advances on the rail (14b) in its side. The cogs (4a, 4b) reaching down under the mouthpiece and the pawl (4c, 4d) at the end of each cog are clearly illustrated in Figure 8a in detail.

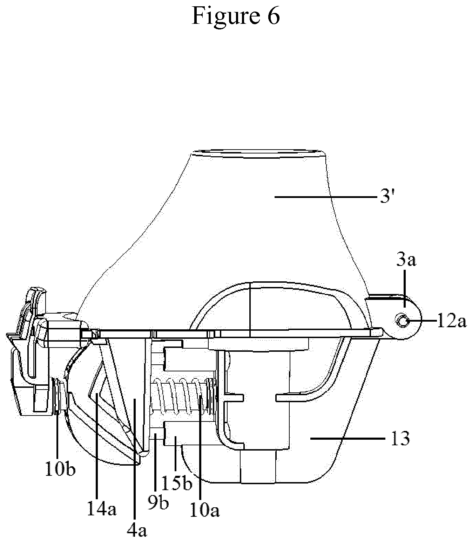

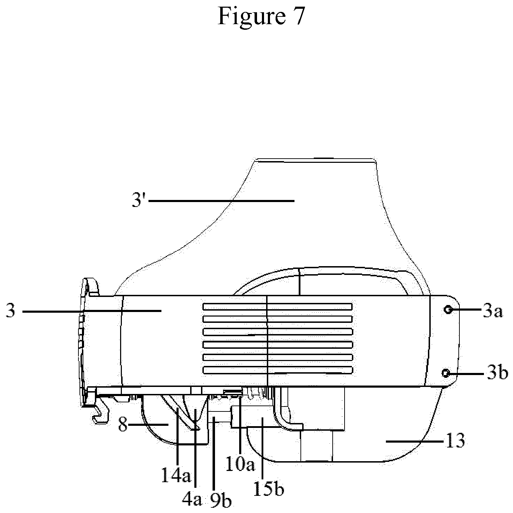

In order to place the capsule containing the medicament in dry powder form into the capsule chamber (13) before inhalation, the mouthpiece (3') which synchronizes with the middle casing (3) has to be moved outwards around the pin (12b) it is joined. This movement of the mouthpiece (3') has to be continued until the mouthpiece (3') gets to a position available to comfortably place the capsule into the capsule chamber (13). So as to pierce the capsule after the capsule is placed into the capsule chamber (13), the route illustrated in figure 5 should be followed while the mouthpiece (3') is closed on the capsule chamber (13). During the movement of the mouthpiece (3') onto the capsule chamber (13), while the pawls (4c, 4d) at the ends of the cogs (4a, 4b) reaching down under the mouthpiece are advancing on the rails (14a, 14b) on both sides of the needle carrier (8), the needle carrier (8) compresses the spring (10a) and the piercing needles (9a, 9b) move towards the capsule. Furthermore, when each of the pawls (4c, 4d) at the ends of the cogs (4a, 4b) reaching down under the mouthpiece gets close to the corner of the rail (14a, 14b) it advances on, the spring (10a) approaches to maximum compression as the needle carrier (8) is moved towards the capsule chamber (13) and the needles (9a, 9b) pierce the capsule passing through the holes on the capsule chamber (15a, 15b). While this movement of the mouthpiece (3') is continuing, the pawls' (4c, 4d) passing the corner causes the spring (10a) to loosen and therefore the needle carrier (8) to move away from the capsule chamber (13). As shown in figure 6 and figure 7, when the mouthpiece (3') is completely closed, the needle carrier (8) is in the accurate and exact position between the two springs (10a, 10b); and the needles (9a, 9b) are out of the capsule. In this position of the mouthpiece (3'), medicament in dry powder form contained in the pierced capsule is ready for inhalation.

The needles (9a, 9b) and needle holes (15a, 15b) mentioned are shown clearly in detail in figure 8. According to this, as the pawls (4c, 4d) at the ends of the cogs (4a, 4b) reaching down under the mouthpiece (3') advance on the rails (14a, 14b) in both sides of the needle carrier (8), the needle carrier (8) moves towards the capsule chamber (13) and the upper needle (9a) passes through the upper needle hole (15a); the lower needle (9b) passes through the lower needle hole (15b) and pierce the capsule in the capsule chamber.

The mouthpiece (3') of the inhaler of the present invention which is joined with the middle casing (3) is illustrated from different angles in figure 8a and figure 8b. The air intake duct (5) advancing into the mouthpiece (3') ensures delivery of the air flow entraining the medicament in dry powder form to the patient.

As it can be seen in figures 9a and 9b, there are three holes ($H_1$, $H_2$, $H_3$) situated at the outlet of the capsule chamber (13), in the area where the capsule chamber is in the center. These holes ($H_1$, $H_2$, $H_3$) are situated at the outlet of the capsule chamber (13), in the area where the capsule chamber is in the center such that there are equal angles between them. In the bottom view of the capsule chamber (13) in figure 9b, two of said holes ($H_1$, $H_2$) can be seen while the other one is not visible in the figure. However, in the plan view of the capsule chamber (13) given in figure 9a, all holes ($H_1$, $H_2$, $H_3$) situated at the outlet of the capsule chamber (13), in the area where the capsule chamber is in the center, can clearly be seen. While some of the external air entering the device upon inhalation of the patient flows into the capsule chamber (13) and entrains the dry powder medicament in the capsule from the capsule and then the capsule chamber (13) towards the mouthpiece (3'), the rest of the air enters through the holes situated at the outlet of the capsule chamber (13), in the area where the capsule chamber is in the center, and creates turbulence in the air flow entraining medicament in dry powder form at the outlet of the capsule chamber (13).

According to figure 2, there is a sieve (6) at the outlet of the capsule chamber (13) in air intake duct (5) side and preferably a sieve holder (7) which keeps this sieve fixed. The air intake duct (5) which is inserted into the mouthpiece (3') also fits into this sieve holder (7). Thus, as the air intake duct (5) is on the same axis with the sieve (6) and the sieve holder (7), the air exiting the capsule chamber (13) passes through the sieve (6), the sieve holder (7) and the air intake duct (5) and reaches the patient.

External air entering the device upon inhalation of the patient goes into the holes on the capsule chamber (13) and entrains the medicament in dry powder form contained in the capsule out. Then, the air flow entraining the medicament in dry powder form ensures delivery of the dry powder medicament to the patient passing through the sieve (6), the sieve holder (7) and the air intake duct (5).

After the medicament in dry powder form in the pierced capsule is inhaled by the patient, the mouthpiece (3') is moved outwards and removed from the capsule chamber (13). The capsule chamber (13) is exposed when the mouthpiece (3') synchronizing with the middle casing (3) is moved outwards around the pin it is joined. Hence, the capsule discharged after inhalation is ejected from the capsule chamber (13) and the capsule chamber (13) is cleaned properly if required. The mouthpiece (3') and the mouthpiece cover (1) are kept close (figure 1) until the following inhalation.

The capsule containing the dry powder medicament in the inhaler of the present invention is produced according to the prior art. According to the present invention, the particle size of the active agents comprised in the dry powder medicament contained in the capsule is less than 20 μm, preferably less than 10 μm.

The inhaler of the present invention has been designed so as to deliver the dry powder medicament used in monotherapy or combined therapy. The term "monotherapy" refers to inhalation treatments in which dry powder medicaments comprising a single active agent are used whereas the term "combined therapy" refers to inhalation treatments in which dry powder medicaments comprising more than one active agent are used.

The dry powder medicament delivered via the device of the present invention comprises at least one excipient in addition to the active agent or agents. These excipients are generally chosen from a group comprising monosaccharides (glucose, arabinose, etc.), disaccharides (lactose, saccharose, maltose, etc.), oligo- and polysaccharides (dextran, etc.), polyalcohols (sorbite, mannite, xylite), salts (sodium chloride, calcium carbonate, etc.) or combinations thereof. According to the present invention, the medicament in dry powder form preferably comprises lactose as the excipient. The medicament in dry powder form comprises fine or coarse excipient particles preferably having various particle size ranges in order to deliver the required amount to the lungs.

The active agent or the active agents comprised in the dry powder medicament which is stored in capsules used in the device of the present invention can be selected from a group comprising cromolyns, anti-infectives, antihistamines, steroids, anti-inflammatories, bronchodilators, leukotirene inhibitors, PDE IV inhibitors, antitussives, diuretics, anticholinergics, hormones, xanthines and pharmaceutically acceptable derivatives thereof.

The active agent comprised in the medicament in dry powder form delivered via the inhaler of the present invention is preferably selected from a group comprising tiotropium, oxitropium, flutropium, ipratropium, glicopironium, flunisolid, beclomethasone, budesonide, fluticasone, mometasone, ciclesonide, rofleponide, dexamethasone, montelukast, methylcyclopropane acetic acid, sodium cromoglicat, nedocromil sodium, Npropylene, teophylline, roflumilast, ariflo (cilomilast), salmeterol, salbutamol, formoterol, terbutaline, carmoterol, indacaterol, cetirizine, levocetirizine, efletirizine, fexofenadine and their racemates, free base, enantiomers or diastereomers and their pharmaceutically acceptable salts, solvates and/or hydrates or a combination of said active agents, for instance dual, triple, quadruple, quinary combinations.

The device of the present invention is used in the administration of the medicament in dry powder form which is utilized in the treatment of respiratory diseases, particularly in asthma, chronic obstructive pulmonary disease (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary including emphysema and chronic bronchitis, airways or lung diseases (COPD, COAD or COLD), pneumoconiosis, aluminosis, anthracosis, asbestosis, chalicosis, ptilosis, siderosis, silicosis, tabacosis and byssinosis. The device of the invention can be used in prophylactic or symptomatic treatment. In addition, the medicament in dry powder form which is preferably used in the symptomatic treatment of allergic asthma and COPD is administered to the patient via the device of the present invention.

The present disclosure also provides:

A. An inhaler comprising capsule which contains medicament in dry powder form comprising;

* a mouthpiece cover (1) covering the mouthpiece (3'),

* a bottom casing (2) wherein the mechanism of the device is situated,

* a movable mouthpiece (3') communicating with the bottom casing of the device,

* a middle casing (3) positioned between the bottom casing (2) and the mouthpiece (3'), a capsule chamber (13) wherein the capsule is placed

characterized in that the ratio of the diameter of the needles (9a, 9b) piercing the capsule containing dry powder medicament to the diameter of the needle holes (15a, 15b) through which said needles pass and enter the capsule chamber (13) in order to pierce the capsule is in the range of 0.1 to 1.0.

B. The inhaler according to clause A, wherein the ratio of the diameter of the needles (9a, 9b) piercing the capsule containing dry powder medicament to the diameter of the needle holes (15a, 15b) through which said needles pass and enter the capsule chamber (13) in order to pierce the capsule is in the range of 0.25 to 1.0.

C. The inhaler according to clause A and B, wherein the ratio of the diameter of the needles (9a, 9b) piercing the capsule containing dry powder medicament to the diameter of the needle holes (15a, 15b) through which said needles pass and enter the capsule chamber (13) in order to pierce the capsule is in the range of 0.3 to 1.0.

D. The inhaler according to clauses A-C, wherein the diameter values of the needle holes (15a, 15b) are identical.

E. The inhaler according to clauses A-C, wherein the diameter values of the needles (9a, 9b) are identical.

F. The inhaler according to clauses A-E, wherein the number of needle holes (15a, 15b) is at least two.

G. The inhaler according to clauses A-F, wherein the needle holes (15a, 15b) are hollow-cylindrical through which the needles (9a, 9b) can pass.

H. The inhaler according to clauses A-G, wherein the needles (9a, 9b) are cylindrical and the points of the needles (9a, 9b) used to pierce the capsule are sharp.

I. The inhaler according to clauses A-H, wherein the needle holes (15a, 15b) can be situated diagonally, symmetrically, asymmetrically, side by side and/or vis-à-vis on a horizontal or vertical line, at equal and/or different intervals.

J. The inhaler according to clauses A-I, wherein the needles (9a, 9b) are made of stainless steel.

K. The inhaler comprising capsule which contains medicament in dry powder form comprising;

- a mouthpiece cover (1) covering the mouthpiece (3'),

- a bottom casing (2) wherein the mechanism of the device is situated,

- a movable mouthpiece (3') communicating with the bottom casing of the device,

- a middle casing (3) positioned between the bottom casing (2) and the mouthpiece (3'),

- a capsule chamber (13) wherein the capsule is placed

characterized in that there are at least two holes (H1, H2 and/or H3) having identical or different diameter values situated at the outlet of the capsule chamber (13), in the area where the capsule chamber (13) is in the center.

L. The inhaler according to clause K, wherein the diameter of each hole (H1, H2 and/or H3) situated at the outlet of the capsule chamber (13), in the area where the capsule chamber is in the center, is in the range of 1 μm to 1000 μm.

M. The inhaler according to clause K and L, wherein the diameter of each hole (H1, H2 and/or H3) situated at the outlet of the capsule chamber (13), in the area where the capsule chamber is in the center is in the range of 1 μm to 750 μm.

N. The inhaler according to clauses K-M, wherein the diameter of each hole (H1, H2 and/or H3) situated at the outlet of the capsule chamber (13), in the area where the capsule chamber is in the center is in the range of 1 μm to 500 μm.

O. The inhaler according to clauses K-N, wherein the diameters of the holes (H1, H2 and/or H3) situated at the outlet of the capsule chamber (13), in the area where the capsule chamber is in the center are preferably identical.

P. The inhaler according to clauses K-N, wherein the diameters of the holes (H1, H2 and/or H3) situated at the outlet of the capsule chamber (13), in the area where the capsule chamber is in the center are preferably different.

Q. The inhaler according to clauses K-P, wherein the number of the holes situated at the outlet of the capsule chamber (13), in the area where the capsule chamber is in the center is at least two.

R. The inhaler according to clauses K-Q, wherein the number of the holes situated at the outlet of the capsule chamber (13), in the area where the capsule chamber is in the center is three.

S. The inhaler according to clauses K-R, wherein the holes (H1, H2 and/or H3) at the outlet of the capsule chamber (13), in the area where the capsule chamber is in the center are situated such that the angles between them are equal.

T. The inhaler according to any of the preceding clauses, wherein the mouthpiece (3') that is exposed when the mouthpiece cover (1) is opened is joined with the middle casing (3), and the mouthpiece (3') and the middle casing (3) synchronize.

U. The inhaler according to clauses A, K and T, wherein the mechanism of the inhaler comprises needles piercing the capsule (9a, 9b), the needle carrier (8) and the springs (10a, 10b).

V. The inhaler according to clauses A, K, T and U, wherein said inhaler further comprises the sieve (6) and a sieve holder (7) positioned above the sieve.

W. The inhaler according to clause V, wherein the air intake duct (5) is inserted into the sieve holder (7).

X. The inhaler according to clause V and W, wherein the air intake duct (5) is on the same axis with the sieve (6) and the sieve holder (7).

**Analysis Results**

Using DUSA (Dosage Unit Sampling Apparatus) device, tables 1-5 illustrate the ratios of the diameters of the needles to the diameters of the needle holes (r) for the flow rate (Q) values required to be applied by the patient during inhalation in order to ensure standard pressure decrease (△P) of 4 kPa in the inhaler of the present invention after the capsule is pierced. The ratio of diameter of the needles to the diameter of the needle holes (r) was kept constant and 10 measurements were conducted for each diameter value. Analysis results under given condition are as follows.

**Table 1: Q and △P values when the ratio of diameter of the needles to the diameter of the needle holes is 0.25**

|  | Q(L/min) | (△P) (kPa) |
|---|---|---|
|  | 33.70 | 4.00 |
|  | 31.20 | 4.00 |
|  | 32.86 | 4.00 |
|  | 33.96 | 4.00 |
|  | 33.58 | 4.00 |
| r = 0.25 | 34.52 | 4.00 |
|  | 31.93 | 4.00 |
|  | 30.92 | 4.00 |
|  | 34.10 | 4.00 |
|  | 28.25 | 4.00 |

(continued)

|  | Q(L/min) | (△P) (kPa) |
|---|---|---|
| Average: | 32.97 | 4.00 |

**Table 2: Q and △P values when the ratio of diameter of the needles to the diameter of the needle holes is 0.30**

|  | Q(L/min) | (△P) (kPa) |
|---|---|---|
|  | 33.94 | 4.00 |
|  | 36.71 | 4.00 |
|  | 34.72 | 4.00 |
|  | 37.32 | 4.00 |
|  | 37.65 | 4.00 |
| r = 0.30 | 37.47 | 4.00 |
|  | 35.83 | 4.00 |
|  | 3.10 | 4.00 |
|  | 36.47 | 4.00 |
|  | 37.84 | 4.00 |
| Average: | 36.61 | 4.00 |

**Table 3: Q and △P values when the ratio of diameter of the needles to the diameter of the needle holes is 0.50**

|  | Q(L/min) | (△P) (kPa) |
|---|---|---|
|  | 37.22 | 4.00 |
|  | 39.81 | 4.00 |
|  | 39.12 | 4.00 |
|  | 39.75 | 4.00 |
|  | 37.93 | 4.00 |
| r = 0.50 | 38.76 | 4.00 |
|  | 38.51 | 4.00 |
|  | 38.89 | 4.00 |
|  | 38.91 | 4.00 |
|  | 39.65 | 4.00 |
| Average: | 38.86 | 4.00 |

**Table 4: Q and ΔP values when the ratio of diameter of the needles to the diameter of the needle holes is 0.70**

|  | Q(L/min) | (ΔP) (kPa) |
|---|---|---|
| r = 0.70 | 40.02 | 4.00 |
|  | 41.75 | 4.00 |
|  | 39.72 | 4.00 |
|  | 39.78 | 4.00 |
|  | 39.15 | 4.00 |
|  | 40.43 | 4.00 |
|  | 39.37 | 4.00 |
|  | 38.83 | 4.00 |
|  | 41.05 | 4.00 |
|  | 41.78 | 4.00 |
| Average: | 40.19 | 4.00 |

**Table 5: Q and ΔP values when the ratio of diameter of the needles to the diameter of the needle holes is 0.90**

|  | Q(L/min) | (ΔP) (kPa) |
|---|---|---|
| r = 0.90 | 43.27 | 4.00 |
|  | 41.42 | 4.00 |
|  | 43.89 | 4.00 |
|  | 41.18 | 4.00 |
|  | 41.51 | 4.00 |
|  | 40.92 | 4.00 |
|  | 41.38 | 4.00 |
|  | 37.12 | 4.00 |
|  | 39.72 | 4.00 |
|  | 40.18 | 4.00 |
| Average: | 41.06 | 4.00 |

**Claims**

1. An inhaler comprising capsule which contains medicament in dry powder form comprising;

   • a mouthpiece cover (1) covering the mouthpiece (3'),
   • a bottom casing (2) wherein the mechanism of the device is situated,
   • a movable mouthpiece (3') communicating with the bottom casing of the device,
   • a middle casing (3) positioned between the bottom casing (2) and the mouthpiece (3'),
   • a capsule chamber (13) wherein the capsule is placed

   **characterized in that** there are at least two holes ($H_1$, $H_2$ and/or $H_3$) having identical or different diameter values situated at the outlet of the capsule chamber (13), in the area where the capsule chamber (13) is in the center.

2. The inhaler according to claim 1, wherein the diameter of each hole ($H_1$, $H_2$ and/or $H_3$) situated at the outlet of the capsule chamber (13), in the area where the capsule chamber is in the center, is in the range of 1 μm to 1000 μm.

3. The inhaler according to claim 1 and 2, wherein the diameter of each hole ($H_1$, $H_2$ and/or $H_3$) situated at the outlet of the capsule chamber (13), in the area where the capsule chamber is in the center is in the range of 1 μm to 750 μm.

4. The inhaler according to claims 1-3, wherein the diameter of each hole ($H_1$, $H_2$ and/or $H_3$) situated at the outlet of the capsule chamber (13), in the area where the capsule chamber is in the center is in the range of 1 μm to 500 μm.

5. The inhaler according to claims 1-4, wherein the diameters of the holes ($H_1$, $H_2$ and/or $H_3$) situated at the outlet of the capsule chamber (13), in the area where the capsule chamber is in the center are preferably identical.

6. The inhaler according to claims 1-4, wherein the diameters of the holes ($H_1$, $H_2$ and/or $H_3$) situated at the outlet of the capsule chamber (13), in the area where the capsule chamber is in the center are preferably different.

7. The inhaler according to claims 1-6, wherein the number of the holes situated at the outlet of the capsule chamber (13), in the area where the capsule chamber is in the center is at least two.

8. The inhaler according to claims 1-7, wherein the number of the holes situated at the outlet of the capsule chamber (13), in the area where the capsule chamber is in the center is three.

9. The inhaler according to claims 1-8, wherein the holes ($H_1$, $H_2$ and/or $H_3$) at the outlet of the capsule chamber (13), in the area where the capsule chamber is in the center are situated such that the angles between them are equal.

10. The inhaler according to any of the preceding claims, wherein the mouthpiece (3') that is exposed when the mouthpiece cover (1) is opened is joined with the middle casing (3), and the mouthpiece (3') and the middle casing (3) synchronize.

11. The inhaler according to claims 1, and 10, wherein the mechanism of the inhaler comprises needles piercing the capsule (9a, 9b), the needle carrier (8) and the springs (10a, 10b).

12. The inhaler according to claims 1, 10 and 11, wherein said inhaler further comprises the sieve (6) and a sieve holder (7) positioned above the sieve.

13. The inhaler according to claim 12, wherein the air intake duct (5) is inserted into the sieve holder (7).

14. The inhaler according to claim 12 and 13, wherein the air intake duct (5) is on the same axis with the sieve (6) and the sieve holder (7).

Figure 1

Figure 2

Figure 3

## Figure 4a

## Figure 4b

Figure 5

# Figure 6

Figure 7

Figure 8

## Figure 8a

## Figure 8b

## Figure 9a

## Figure 9b

# Figure10

1
5
7
3c
6
3d
11
13
2
10b  9a  9b  10a

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2008 014025 A1 (BOEHRINGER INGELHEIM PHARMA [DE]) 17 September 2009 (2009-09-17) * paragraphs [0012], [0013]; figure 1 * | 1-14 | INV. A61M15/00 |
| X | WO 02/083220 A2 (ADVANCED INHALATION RES INC [US]) 24 October 2002 (2002-10-24) * The whole document, especially Fig. 2,3 and 7B * | 1-11,14 | |
| X | CN 2 753 402 Y (SHANGHAI PHARMACEUTICAL INDUST [CN]) 25 January 2006 (2006-01-25) * figures 1,3,4 * | 1-11,14 | |

TECHNICAL FIELDS
SEARCHED      (IPC)

A61M

The present search report has been drawn up for all claims

| Place of search | Date of completion of the search | Examiner |
|---|---|---|
| Munich | 11 September 2017 | Borowski, Aleksander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 17 1252

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2017

| Patent document cited in search report | Publication date | Patent family member(s) | Publication date |
|---|---|---|---|
| DE 102008014025 A1 | 17-09-2009 | CA 2717275 A1 | 17-09-2009 |
| | | CA 2838366 A1 | 17-09-2009 |
| | | DE 102008014025 A1 | 17-09-2009 |
| | | DK 2254634 T3 | 12-08-2013 |
| | | EP 2254634 A1 | 01-12-2010 |
| | | EP 2433665 A2 | 28-03-2012 |
| | | ES 2412754 T3 | 12-07-2013 |
| | | JP 5649980 B2 | 07-01-2015 |
| | | JP 5668024 B2 | 12-02-2015 |
| | | JP 2011513012 A | 28-04-2011 |
| | | JP 2012196495 A | 18-10-2012 |
| | | US 2011120465 A1 | 26-05-2011 |
| | | WO 2009112521 A1 | 17-09-2009 |
| WO 02083220 A2 | 24-10-2002 | AT 316397 T | 15-02-2006 |
| | | CA 2444129 A1 | 24-10-2002 |
| | | CY 1108214 T1 | 12-02-2014 |
| | | DE 60208891 T2 | 05-10-2006 |
| | | DK 1381416 T3 | 29-05-2006 |
| | | EP 1381416 A2 | 21-01-2004 |
| | | ES 2256467 T3 | 16-07-2006 |
| | | HK 1061658 A1 | 08-09-2006 |
| | | IL 158357 A | 28-02-2013 |
| | | JP 2004526523 A | 02-09-2004 |
| | | MX PA03009412 A | 07-03-2005 |
| | | NZ 528682 A | 25-02-2005 |
| | | PL 204303 B1 | 31-12-2009 |
| | | US 2003150453 A1 | 14-08-2003 |
| | | US 2004011360 A1 | 22-01-2004 |
| | | US 2004154618 A1 | 12-08-2004 |
| | | US 2004154619 A1 | 12-08-2004 |
| | | US 2004216738 A1 | 04-11-2004 |
| | | US 2006283448 A1 | 21-12-2006 |
| | | WO 02083220 A2 | 24-10-2002 |
| CN 2753402 Y | 25-01-2006 | NONE | |

EPO FORM P0459

For more details about this annex : see Official Journal of the European Patent Office, No. 12/82